# EUROPEAN PATENT APPLICATION

(11) **EP 1 892 248 A1**
(43) Date of publication of application: **27.02.2008**
(21) Application number: 06017336.6
(22) Date of filing: 21.08.2006
(51) Int. Cl.: C07K 14/435

(54) **Specific and high affinity binding proteins comprising modified SH3 domains of FYN kinase**

(71) Applicant: Eidgenössische Technische Hochschule Zürich, 8092 Zürich (CH)
(72) Inventor: Grabulovski, Dragan, 8405 Winterthur (CH)
(74) Representative: Kasche, André

(57) **Abstract**

The present invention relates to a recombinant binding protein comprising at least one derivative of the Src homology 3 domain (SH3) of the FYN kinase, wherein at least one amino acid in or positioned up to two amino acids adjacent to the src loop and/or at least one amino acid in or positioned up to two amino acids adjacent to the RT loop is substituted, deleted or added. Furthermore, the Invention is directed to fusion proteins comprising a binding protein according to the invention fused to a pharmaceutically and/or diagnostically active component. In addition, the invention concerns nucleotides coding for these binding and/or fusion proteins as well as corresponding vectors and host calls. Moreover, the invention also concerns a method for co-crystallizing said binding and corresponding target proteins and a method for purifying and/or enriching proteins by contacting a binding protein of the invention with a corresponding target protein. Last but not least, the present invention relates to the use of binding and/or fusion proteins of the present invention for preparing a medicament or a diagnostic means as well as to pharmaceutical or diagnostic compositions comprising said binding and/or fusion proteins.

SEQUENCE LISTING

<110> Eidgenoessische Technische Hochschule Zuerich

<120> Specific and high affinity binding proteins comprising modified
SH3 domains of FYN kinase

<130> 50027EP

<160> 3

<170> PatentIn version 3.3

<210> 1
<211> 63
<212> PRT
<213> Homo sapiens

<400> 1

Gly Val Thr Leu Phe Val Ala Leu Tyr Asp Tyr Glu Ala Arg Thr Glu
1               5                   10                  15

Asp Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 2
<211> 63
<212> PRT
<213> Artificial

<400> 2

Gly Val Thr Leu Phe Val Ala Leu Tyr Asp Tyr Glu Ala Ile Thr Glu
1               5                   10                  15

Asp Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 3
<211> 65
<212> PRT
<213> Artificial

<220>
<223> Variant of SH3 domain of Fyn kinase with high affinity to ED-B
domain of fibronectin

<400> 3

Gly Val Thr Leu Phe Val Ala Leu Tyr Asp Tyr His Ala Gln Ser Gly
1               5                   10                  15

Ala Asp Leu Ser Phe His Lys Gly Glu Lys Phe Gln Ile Leu Lys Phe
            20                  25                  30

Gly Arg Gly Lys Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

## Description

### Field of the invention

The present invention relates to a recombinant binding protein comprising at least one derivative of the Src homology 3 domain (SH3) of the FYN kinase, wherein at least one amino acid in or positioned up to two amino acids adjacent to the src loop and/or at least one amino acid in or positioned up to two amino acids adjacent to the RT loop is substituted, deleted or added. Furthermore, the invention is directed to fusion proteins comprising a binding protein according to the Invention fused to a pharmaceutically and/or diagnostically active component. In addition, the invention concerns nucleotides coding for these binding and/or fusion proteins as well as corresponding vectors and host cells. Moreover, the invention also concerns a method for co-crystallizing said binding and corresponding target proteins and a method for purifying and/or enriching proteins by contacting a binding protein of the invention with a corresponding target protein. Last but not least, the present Invention relates to the use of binding and/or fusion proteins of the present invention for preparing a medicament or a diagnostic means as well as to pharmaceutical or diagnostic compositions comprising said binding and/or fusion proteins.

### Background of the Invention

Specific and high-affinity binding agents are indispensable tools for biological and medical research and also have utility for medical diagnosis, prophylaxis and treatment. At present, monoclonal antibodies are the predominant class of binding molecules that can be rapidly isolated with high affinity and specificity to virtually any target. However, immunoglobulins have limitations that are based mostly on their general biophysical properties and their rather complicated molecular structure. Therefore, already in the 1990's several research groups have explored small globular proteins as substitutes for antibodies. The idea behind this concept is the transfer of a universal binding site from an antibody structure to alternative protein frameworks, the so-called scaffolds. So far more than 40 scaffolds have been described, among them two SH3 domains, the SH3 domains of the Abl and the Src kinase (see Binz et al., Nature Biotechnology, Vol. 23, No. 10, 1257-1268, 2005).

SH3 domains are found in many different proteins involved in intracellular signalling and cytoskeletal organization (Cohen et al., "Modular binding domains In signal transduction proteins." Cell 80(2): 237-48, 1995). Despite the variability in their primary structures these SH3 domains share a very similar overall structure and mode of binding to proteins sharing the minimal consensus sequence PxxP that is a critical determinant for natural SH3 binding. An Important function of SH3 domains is to participate In highly selective protein-protein interactions.

Erpel et al. ("Mutational analysis of the Src SH3 domain: the same residues of the ligand binding surface are Important for intra- and intermolecular interactions." Embo J. 14(5): 963-75,1995) Investigated the influence of mutations in the RT and n-Src loops of Src SH3 domains and demonstrated that mutations in both loops which are adjacent to the hydrophobic surface could Influence the ability of this domain to participate in inter- and intramolecular associations.

Hilpakka et al. ("SH3 domains with high affinity and engineered ligand specificities targeted to HIV-1 Nef." J. Mol. Biol. 293(5): 1097-106, 1999) investigated the ability of the RT-loop of the Hck SH3 domain to act as a versatile specificity and affinity determinant. The authors constructed a phage library of Hck domains, where 6 amino acids of the RT-Loop were randomized (termed RRT-SH3). Using this strategy they identified individual RRT-SH3 domains that can bind to HIV-1 Nef up to 40 times better than Hck-Sh3. The authors indicate the importance of the RT loop In SH3 ligand selection as a general strategy for creating SH3 domains with desired binding properties.

Lee et al. ("A single amino acid in the SH3 domain of Hck determines Its high affinity and specificity In binding to HIV-1 Nef protein." Embo. J. 14(20): 5006-15, 1995) Investigated the structural basis of the different SH3 binding affinities and specificities of Hck to the HIV-1 Nef protein and were able to transfer the binding property of Hck SH3 towards Nef to the Fyn SH3 domain by a single mutation in the RT loop of the Fyn SH3 domain (R961).

Hosse et al. ("A new generation of protein display scaffolds for molecular recognition", Protein Science, 15:14-27, 2006) specifically address the requirements for binding proteins suitable for therapeutic applications. The authors note the importance of some characteristics for therapeutically useful binding proteins such as serum stability, tissue penetration, blood clearance, target retention and Immune response. In the latter respect it is noted that non-human therapeutic proteins should be made as similar to their human counterparts as possible and a human scaffold might be less immunogenic right from the start. These authors conclude:
"However, even an entirely human scaffold is no guarantee for a protein that does not elicit a human immune response, especially If it is an intracellular protein. Randomization of amino acids during library construction can potentially introduce novel T-cell epitopes. Even single point mutations can render a human protein immunogenic. Furthermore, most human scaffolds cause some autoimmune response."

Today, the SH3 domains of Abl and Hck kinases are acknowledged as protein scaffolds for generating protein binders with prescribed specificity, even though only binders towards known ligands like the Nef proteins or synthetic peptides have been identified so far (see Binz et al. above).

The SH3 domain of the Fyn kinase (Fyn SH3) comprises 63 residues (aa 83-145 of the sequence reported by Semba et al. ("yes-related protooncogene, syn, belongs to the protein-tyrosine kinase family." Proc. Natl. Acad. Sci. U S A 83(15): 5459-63, 1986) and Kawakami et al. ("Isolation and oncogenic potential of a novel human src-like gene." Mol Cell Biol. 6(12): 4195-201, 1986). Fyn is a 59 kDa member of the Src family of tyrosine kinases. As a result of alternative splicing the Fyn protein exists in two different isoforms differing in their kinase domains: one form is found in thymocytes, splenocytes and some hematolymphoid cell lines, while a second form accumulates principally in brain (Cooke and Perlmutter, "Expression of a novel form of the Fyn proto-oncogene in hematopoietic cells." New Biol. 1(1): 66-74, 1989). The biological functions of Fyn are diverse and include signalling via the T cell receptor, regulation of brain function as well as adhesion mediated signalling (Resh, M. D. "Fyn, a Src family tyrosine kinase." Int. J. Biochem. Cell Biol. 30(11): 1159-62, 1998). It Is an intracellular protein. SEQ ID NO: 1 shows the Fyn SH3 sequence (aa 83-145 of Fyn kinase as reported by Kawakami et al. and Semba et al. in 1986, see above):

GVTLFVALYDYEARTEDDLSFHKGEKFQILNSSEGDWWEARSLTTGETGYIPSNY
VAPVDSIQ (SEQ ID NO: 1)

The sequence of the RT-Src and the n-Src loop are underlined and double-underfined, respectively.

The amino acid sequence of Fyn SH3 is fully conserved among man, mouse, rat and monkey (gibbon), Chicken Fyn SH3 differs in one, the one of *Xenopus laevis* in two amino acid positions from the corresponding human domain. Just as other SH3 domains the Fyn SH3 is composed of two antiparallel β-sheets and contains two flexible loops (called RT-Src and n-Src-loops) In order to interact with other proteins.

In summary, the prior art teaches protein frameworks, the so-called scaffolds, as alternatives to established antibody structures. The Src homology 3 domain (SH3) is one of these about 40 or more scaffolds. Among the many different SH3 domains (about 300 In the human genome and several thousands described so far in nature) the Fyn SH3 is one, which has been used once before in order to elucidate SH3 binding specificity and affinity in general. The skilled person is also aware that intracellular proteins are particularly prone to produce immune responses and, therefore, are typically less useful or even useless for in *vivo* applications like therapy and diagnosis.

The object underlying the present invention is to provide improved target specific and high affinity binding proteins that are suitable as research, and in particular, as diagnostic and medical agents. Furthermore, these binding proteins should be stable and soluble under physiological conditions, elicit little or no immune effects in humans receiving these, and provide a binding structure that is also accessible by large target structures, i.e. that is not masked by steric hindrance.

### Description of the invention

It was surprisingly found that the SH3 domain of the Fyn kinase of the Src family provides excellent properties for designing recombinant binding domains with specificity and high affinity to selected targets. In particular, it was found that the target specificity can be designed by mutating the RT loop and/or the src loop resulting in higher variability and improved binding properties for many targets.

Moreover, It was unexpectedly found that not only the native Fyn SH3 binding protein but also mutated Fyn SH3-derived binding proteins were not immunogenic *in vivo.* Therefore, recombinant mutant Fyn SH3 binding proteins are particularly useful for the development of non-immunogenic protein therapeutics and/or diagnostics.

As a result of the above, a first aspect the present invention relates to a recombinant binding protein comprising at least one derivative of the Src homology 3 domain (SH3) of the Fyn kinase, wherein
(a) at least one amino acid in or positioned up to two amino acids adjacent to the src loop and/or
(b) at least one amino acid In or positioned up to two amino acids adjacent to the RT loop
Is substituted, deleted or added, wherein the SH3 domain derivative has an amino acid sequence having at least 70, preferably at least 80, more preferably at least
90 and most preferred at least 95 % sequence identity to the amino acid sequence of SEQ ID NO: 1,
with the proviso that the recombinant binding protein does not comprise the amino acid sequence of SEQ ID NO: 2,
and that the recombinant protein Is not a natural SH3 domain containing protein existing in nature.

The amino acid sequence of SEQ ID NO: 2 (the Fyn SH3 variant R961 of Lee et al., see above) is provided below.

GVTLFVALYDYEAITEDDLSFHKGEKFQILNSSEGDWWEARSLTTGETGYIPSNYV
APVDSIQ (SEQ ID NO: 2)

In the context of this invention the RT loop of the Fyn kinase (sometimes also designated RT-Src-loop) consists of the amino acids E A R T E D that are located in positions 12 to 17 In SEQ ID NO: 1. The positions to be substituted, deleted and/or added, i.e. to be mutated, in or adjacent to the RT loop are amino acids 10 to 19, preferably 11 to 18, more preferably 12 to 17.

In the context of this invention the src loop of the FYN kinase (sometimes also designated n-Src-loop) consists of the amino acids N S S E that are located in positions 31 to 34 in SEQ ID NO: 1. The positions to be substituted, deleted and/or added, i.e. to be mutated, in or adjacent to the src loop are amino acids 29 to 36, preferably 30 to 35, more preferably 31 to 34.

The recombinant protein of the Invention Is not a natural SH3 domain containing protein existing In or Isolated from nature. In other words, the scope of the invention excludes wild type SH3 domain containing proteins. There are abundant SH3 domain containing proteins in nature. These natural SH3 proteins have a binding affinity to their natural ligands. Most if not all of these natural SH3 ligands have a PxxP motif. However, the recombinant proteins of the invention are engineered proteins designed for having affinities to non-natural targets, i.e. non-natural targets being any target, e.g. in nature, preferably in a mammalian, more preferably in a human, excluding natural (wild-type) SH3 ligands. More preferably, the recombinant proteins of the invention essentially have no binding affinity to any natural SH3 binding ligands, most preferably not to any natural SH3 binding ligand having a PxxP motif.

Preferably, the number of amino acids to be added into one and/or both loops is 1 to 20, more preferably 1 to 10 or 1 to 5 amino acids, and most preferably no amino acids are added Into the loops.

In another preferred embodiment, the portions of the SH3 domain derivative that lie outside the RT and src loops are conserved as much as possible in order not to introduce Immunogenic motifs.

It is preferred that the recombinant proteins of the invention essentially do not elicit an Immunogenic reaction In mammals, preferably in mouse, rat and/or human, most preferably in human. Of course, the immunogenicity of the complete recombinant protein of the Invention will not only depend on the SH3 domain derivative portion but can be influenced by other portions of the whole protein.

In a preferred embodiment of the invention, at least the SH3 domain derivative portion of the recombinant protein is essentially non-immunogenic in mammals, preferably In mouse, rat and/or human, most preferably in human.

For example, the person skilled in the art can determine immunogenic reactions of the recombinant protein or its SH3 domain derivative portion by standard and routine techniques, e.g. by administering (e.g. I.v. injection) a recombinant protein of interest or Its SH3 domain derivative to a mammal such as a mouse and analysing the response of immunogenic blood cells and/or factors (e.g. interleukins) after an appropriate time for an immune reaction to occur.

In a more preferred embodiment the binding protein according to the invention Is one, wherein said SH3 domain derivative has at least 70 or at least 85, preferably at least 90, more preferably at least 95, most preferably at least 98 to 100 % Identity to the Src homology 3 domain (SH3) of the FYN kinase outside the src and RT loops.

In a preferred embodiment mutations are introduced in both the RT and src loops.

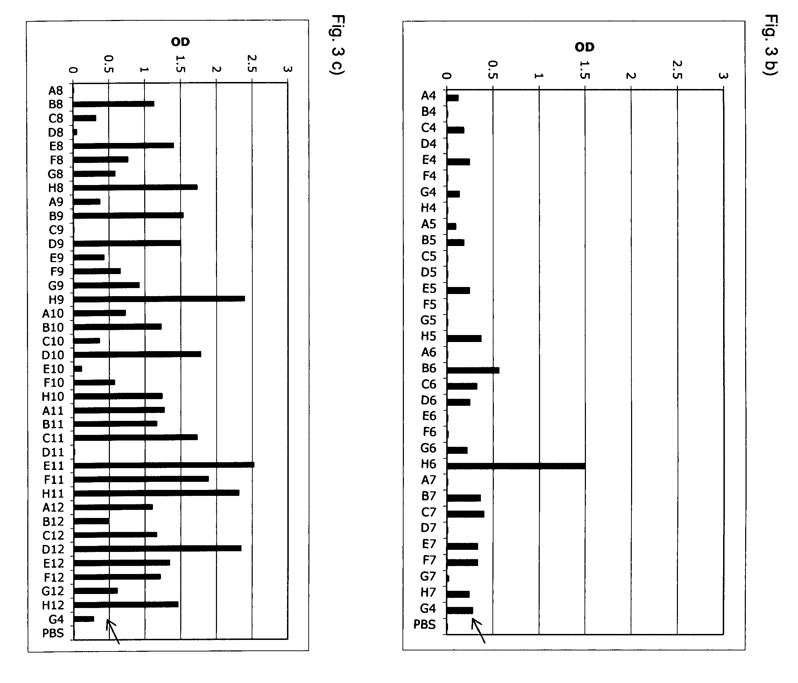

In a further more preferred embodiment the binding protein of the invention comprises one or preferably two altered residues in positions 37 and/or 50 of the SH3 domain derivative, preferably two hydrophobic altered residues, more preferably Trp37 and/or Tyr50, Trp37 and Tyr50 being most preferred. As demonstrated in figure 3b below their randomization can increase the affinity.

The term "derivative of the Src homology 3 domain (SH3) of the FYN klnase", as It Is used herein, is meant to encompass an amino acid sequence having at least 70, preferably at least 80, more preferably at least 90 and most preferred at least 95 % sequence identity to the amino acid sequence of SEQ ID NO: 1. The same meaning holds true for an SH3 domain derivative having at least 70 or at least 85, preferably at least 90, more preferably at least 95, most preferably at least 98 % identity to the Src homology 3 domain (SH3) of the FYN kinase outside the src and RT loops, except that the amino acids forming said loops are excluded when determining the sequence identity.

For the purpose of determining the extent of sequence identity of a derivative of the Fyn SH3 domain to the amino acid sequence of SEQ ID NO: 1, for example, the SIM Local similarity program can be employed (Xiaoquin Huang and Webb Mllier, "A Time-Efficient, Linear-Space Local Similarity Algorithm." Advances in Applied Mathematics, vol. 12: 337-357, 1991.), freely available from the authors and their Institute (see also the world wide web: http://www.expasy.org/tools/sim-prot.html); for multiple alignment analysis ClustalW can be used (Thompson et al.," CLUSTAL W: improving the sensitivity of progressive multiple sequence alignment through sequence weighting, position-specific gap penalties and weight matrix choice.", Nucleic Acids Res., 22(22): 4673-4680, 1994.). Preferably, the extent of the sequence identity of the derivative to SEQ ID NO: 1 Is determined relative to the complete sequence of SEQ ID NO: 1.

In a preferred embodiment the binding protein of the invention comprises at least two derivatives of the Fyn SH3 domain. More preferably, it is a bivalent binding protein. The at least two derivatives of the SH3 domain may be the same or different. Preferably, they are the same.

The binding protein of the invention can be designed to have any specific binding affinity to a given target. In a preferred embodiment, the target is an amino acid-based target such as a peptide or protein, more preferably one comprising a PxxP motif. Of course, only a minority of natural and physiologically relevant target proteins contains a PxxP motif. The examples below demonstrate that binding proteins according to the invention for targets (e.g. ED-B domain of fibronectin) with motifs other than PxxP are available. Therefore, the binding protein of the Invention is by no means limited to the PxxP motif and can have a specific binding affinity to any given target, e.g. sugars, polypeptides, etc.

More preferably, the binding protein according to the invention has a specific binding affinity to a target of 10⁻⁷ to 10⁻¹² M, preferably 10⁻⁸ to 10⁻¹² M, preferably a therapeutically and/or diagnostically relevant target, more preferably an amino acid-based target comprising a PxxP motif.

In a most preferred embodiment, the binding protein according to the invention has a specific (*in vivo* and/or *in vitro*) binding affinity of 10⁻⁷ to 10⁻¹² M, preferably 10⁻⁸ to 10⁻¹² M, to the extra domain of oncofetal fibronectin (ED-B).

Preferably, said ED-B-specific binding protein has one or more, preferably two, altered, preferably hydrophobic, residues In positions 37 and/or 50 of the SH3 domain derivative, in particular Trp37 and/or Tyr50, Trp37 and Tyr50 being most preferred.

Next to a specific binding affinity to polypeptide and protein targets, the binding protein of the invention can also have a specific binding affinity to a small organic or non-aminoacid based compound, e.g. a sugar, oligo- or polysaccharide, fatty acid, etc.

A number of antibody-cytokine fusion proteins have already been investigated for applications in, e.g. arthritis or cancer therapy, often with impressive results. For example, the human antibody L19 specific to the ED-B domain of fibronectin (a marker of angiogenesis) has been used to deliver pro-inflammatory cytokines (such as IL-2, IL-12 or TNF) to solid tumours, sometimes with striking therapeutic benefits [for a review and corresponding references see Neri & Blcknell, Nat. Rev. Cancer (2005) 5: 436-446, and also WO 01/62298].

The binding protein of the present invention now allows for substituting antibodies in prior art fusion proteins and also for designing new and less immunogenic fusion proteins for *in vivo* and *in vitro* pharmaceutical and diagnostic applications.

In a second aspect, the Invention relates to a fusion protein comprising a binding protein of the invention fused to a pharmaceutically and/or diagnostically active component.

A fusion protein of the invention may comprise non-polypeptide components, e.g. non-peptidic linkers, non-peptidic ligands, e.g. for therapeutically or diagnostically relevant radionuclides.

Preferably, said active component is a cytokine, preferably a cytokine selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-Ilgand, TGF-beta, IL-1alpha and IL-1beta.

More preferably, said active component is a toxic compound, preferably a small organic compound or a polypeptide, preferably a toxic compound selected from the group consisting of calicheamicin, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin.

In another preferred embodiment, the fusion protein according to invention is one, wherein said active component is a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MP1F-1, 6Ckine, CTACK, MEC, Lymphotactin and Fractalkine.

In a further preferred embodiment the binding protein according to the invention contains artificial amino acids.

In further preferred embodiments of the fusion protein of the present invention said active component is a fluorescent dye, preferably a component selected from the groups of Alexa Fluor or Cy dyes (Berlier et al., "Quantitative Comparison of Long-wavelength Alexa Fluor Dyes to Cy Dyes: Fluorescence of the Dyes and Their Bioconjugates", J Histochem Cytochem. 51 (12): 1699-1712, 2003.); a photosensitizer, preferably bis(triethanolamine)Sn(IV) chlorin e₆ (SnChe₆); a pro-coagulant factor, preferably tissue factor; an enzyme for pro-drug activation, preferably an enzyme selected from the group consisting of carboxy-peptidases, glucuronidases and glucosidases; a radionuclide either from the group of gamma-emitting isotopes, preferably ^{99m}Tc, ¹²³I, ¹¹¹In, or from the group of positron emitters, preferably ¹⁸F, ⁶⁴Cu, ⁶⁸Ga, ⁸⁶Y, ¹²⁴I, or from the group of beta-emitter, preferably ¹³¹I, ⁹⁰Y, ¹⁷⁷Lu, ⁶⁷Cu, or from the group of alpha-emitter, preferably ²¹³Bi, ²¹¹At; and/or a functional Fc domain, preferably a human functional Fc domain.

The above mentioned functional Fc domain will allow for directing a mammal's immune response to a site of specific target binding of the binding protein component of the fusion protein, e.g. in therapeutic, prophylactic and/or diagnostic applications.

A further preferred embodiment relates to fusion proteins according to the invention as mentioned above, further comprising a component modulating serum half-ilfe, preferably a component selected from the group consisting of polyethylene glycol (PEG), immunoglobulin and albumin-binding peptides.

In a most preferred embodiment, the fusion protein of the invention as mentioned above comprises a binding protein of the invention having a specific (*in vivo* and/or *in vitro*) binding affinity of 10⁻⁷ to 10⁻¹² M, preferably 10⁻⁸ to 10⁻¹² M, to the extra domain of oncofetal fibronectin (ED-B). Preferably, said ED-B-specific binding protein has one or more, preferably two hydrophobic residues in positions 37 and/or 50 of the SH3 domain derivative, in particular Trp37 and/or Tyr50, Trp37 and Tyr50 being most preferred.

Binding and fusion proteins according to the invention may be prepared by any of the many conventional and well known techniques such as plain organic synthetic strategies, solid phase-assisted synthesis techniques or by commercially available automated synthesizers. On the other hand, they may also be prepared by conventional recombinant techniques alone or in combination with conventional synthetic techniques.

Further aspects of the present invention are directed to (i) a polynucleotide coding for a binding protein or fusion protein according to the invention, (ii) a vector comprising said polynucleotide, (iii) a host cell comprising said polynucleotide and/or said vector.

Polynucleotides can be DNA, RNA, PNA and any other analogues thereof. The vectors and host cells may be any conventional type that fits the purpose, e.g. production of binding and fusion proteins of the Invention, therapeutically useful vectors and host cells, e.g. for gene therapy, The skilled person will be able to select those polynucleotides, vectors and host cells from an abundant prior art and confirm their particular suitability for the desired purpose by routine methods and without undue burden.

Moreover, another aspect of the present invention relates to a method for co-crystallizing proteins, wherein a binding protein of the invention is brought Into contact with a second protein to be investigated, crystallized, etc. under conditions that allow for the crystallization of both proteins.

Also, another aspect of the present invention concerns a method for purifying and/or enriching proteins, wherein a binding protein of the invention is contacted with a target protein under conditions that allow for the binding of the binding protein to the target protein.

The binding and fusion proteins of the present Invention do not elicit a strong and preferably have essentially no immune response in mammals, in particular in humans and mice, as was demonstrated for mice and Is analogously expected to hold true for humans, too, because the Fyn SH3 is identical in both mammalian species. It was surprisingly found that neither native Fyn SH3 nor mutated Fyn SH3 causes an immune response in mice injected i.v. with either one. This was unexpected because Fyn kinase Is an Intracellular protein and does not participate in neonatal B cell selection. Therefore, Fyn SH3-derived binding and fusion proteins with designed target specificity and affinity are particularly well suited for therapeutic, prophylactic and/or diagnostic applications in *vivo.*

Hence, a highly relevant aspect of the present invention relates to the use of a binding or fusion protein according to the Invention for preparing a medicament.

In a further aspect, the binding or fusion protein of the invention is used for preparing a diagnostic means, in particular for in *vivo* applications.

Preferably, an ED-B specific binding or fusion protein as described above is used for preparing a medicament or diagnostic means for the treatment or diagnosis of cancer.

Another aspect of the present invention relates to a pharmaceutical composition comprising a binding or fusion protein of the invention and optionally a pharmaceutically acceptable excipient.

Another aspect of the present invention relates to a diagnostic composition, preferably for *in vivo* applications, comprising a binding or fusion protein of the invention and optionally a pharmaceutically acceptable excipient.

Preferably, the pharmaceutical or diagnostic composition comprises an ED-B specific binding or fusion protein of the Invention and optionally a pharmaceutically acceptable excipient.
Pharmaceutical compositions and diagnostic means for in *vivo* applications of the present invention typically comprise a therapeutically or diagnostically effective amount of a binding and/or fusion protein according to the invention and optionally auxiliary substances such as pharmaceutically acceptable excipient(s). Said pharmaceutical compositions are prepared in a manner well known In the pharmaceutical art. A carrier or excipient may be a liquid material which can serve as a vehicle or medium for the active Ingredient. Suitable carriers or excipients are well known In the art and include, for example, stabilizers, antioxidants, pH-regulating substances, controlled-release excipients. The pharmaceutical preparation of the invention may be adapted, for example, for parenteral use and may be administered to the patient In the form of solutions or the like.

Finally, another aspect of the present invention concerns a method of treatment or diagnosis, wherein an effective amount of the above pharmaceutical or diagnostic composition is administered to a patient in need thereof, preferably a patient suffering or suspected of suffering from cancer and/or inflammatory diseases.

In effecting treatment or diagnosis of a subject suffering from diseases, a binding or fusion protein of the present invention can be administered in any form or mode which makes the therapeutic or diagnostic compound bioavailable in an effective amount, Including oral or parenteral routes. For example, compositions of the present invention can be administered subcutaneously, intramuscularly, intravenously and the like. One skilled in the art in the field of preparing formulations can readily select the proper form and mode of administration depending upon the particular characteristics of the product selected, the disease or condition to be treated or diagnosed, the stage of the disease or condition and other relevant circumstances (see. e.g. Remington's Pharmaceutical Sciences, Mack Publishing Co. (1990)). The compositions of the present invention can be administered alone or in the form of a pharmaceutical or diagnostic preparation in combination with pharmaceutically acceptable carriers or excipients, the proportion and nature of which are determined by the solubility and chemical properties of the product selected, the chosen route of administration and standard pharmaceutical and diagnostic practice. The products of the present invention, while effective themselves, may be formulated and administered in the form of their pharmaceutically acceptable salts, such as acid addition salts or base addition salts, for purposes of stability, convenience of crystallization, increased solubility and the like.

### Figures

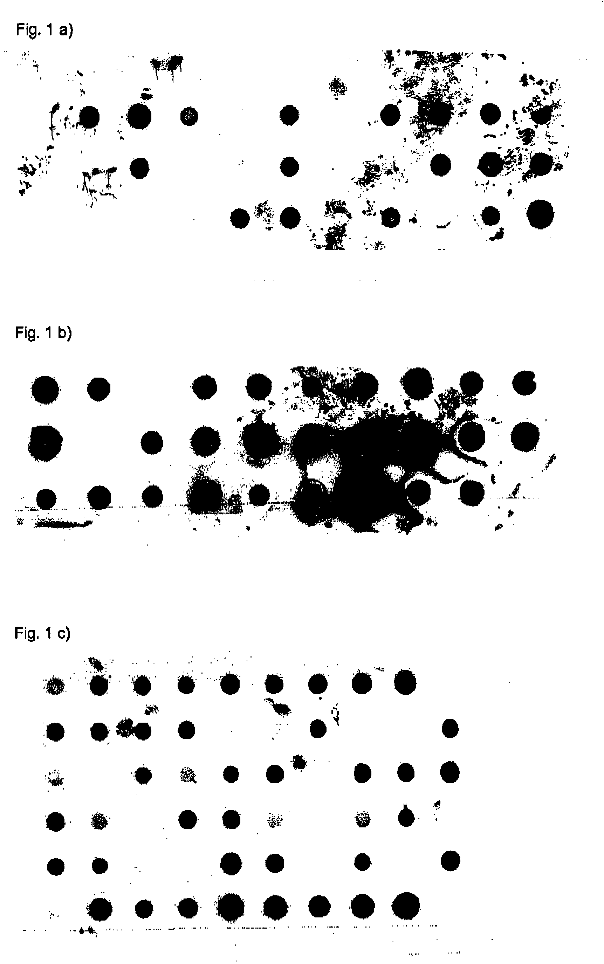

**Fig. 1** Illustrates a dot blot analysis. The percentage of clones expressing a detectable amount of soluble Fyn SH3 mutants was determined by dot blot analysis of bacterial cell lysates using antl-HIS-HRP antibody conjugate (Sigma) as detecting reagent. Peroxidase activity was detected using the ECL plus Western blotting detection system (Amersham).
   A) FynSH3 mutants with randomized RT-Sro-loop.
   B) FynSH3 mutants with an extended (4->6) and randomized n-Src-loop.
   C) FynSH3 with RT- and n-Src randomized loops.

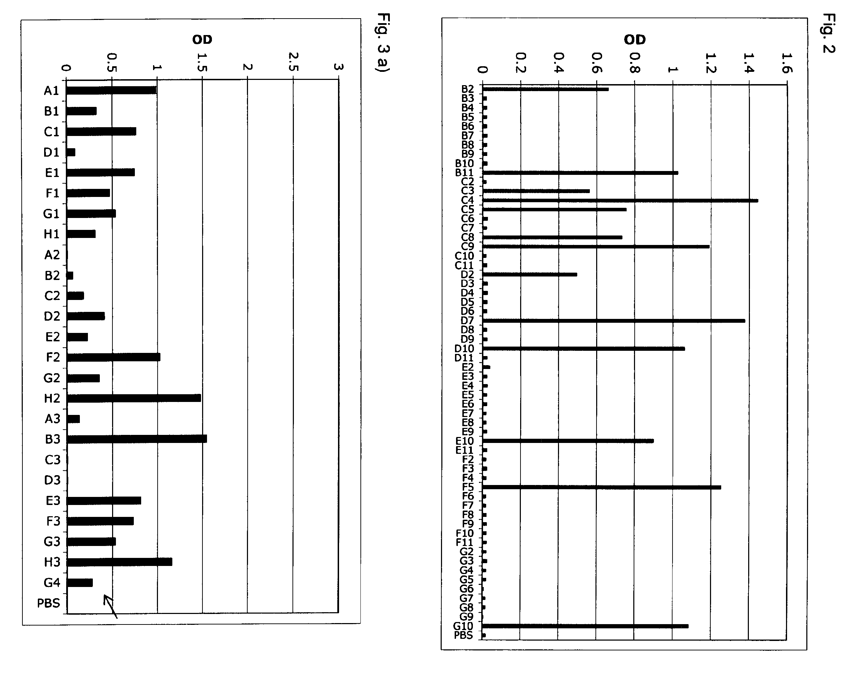

**Fig. 2** illustrates a monoclonal phage-ELISA. After the third round of panning against MSA monoclonal bacterial supernatants containing phages displaying Fyn SH3 mutants were tested by ELISA using MaxlSorp plates (Nunc) coated with MSA (100 µg/ml overnight, 100 µl per well). Bound phages were detected using anti M-13-HRP antibody conjugates (Amersham).
**Fig. 3** illustrates monoclonal phage-ELISA (against MSA) after one round of affinity maturation selection using MaxiSorp plates (Nunc) coated with MSA (100µg/ml overnight, 100 µl per well)
   A) Phage ELISA of the first sub-library of G4 (randomized n-Src loop and Trp37 and Tyr50). The parental clone G4 is indicated with an arrow.
   B) Phage ELISA of the second sub-library of G4 (randomized and extended n-Src loop). The parental clone G4 is indicated with an arrow.
   C) Phage ELISA of the first and second sub-library after one round of panning, performed under conditions favouring binders with a long k_{off}. The parental clone G4 is indicated with an arrow.
**Fig. 4** shows the soluble ELISA (using MaxISorp plates (Nunc) coated with MSA (100 µg/ml overnight, 100 µl per well) of several MSA binding clones, after cloning (pQE-12 vector), expression and purification of the soluble protein, according to the manufacturer's instructions (Qiagen, native conditions). As detecting agents anti-HIS-HRP antibody conjugates were used. As a control the same binding proteins were added to wells blocked with 4% MPBS only.
**Fig. 5** Specificity ELISA of soluble protein. Selected MSA binding Fyn SH3 mutants were tested for binding against human serum albumin (HSA), rat serum albumin (RSA), bovine serum albumin (BSA) and ovalbumin using MaxiSorp plates (Nunc) coated with the different albumins (each 100 µg/ml overnight, 100 µl per well).
**Fig. 6** BIACore analysis of D3. Used concentrations; 4, 2, 1, and 0,5 µM (from top).
**Fig. 7** ELISA analysis of blood samples for the presence of murine antibodies.
   A) MaxiSorp plates (Nunc) were coated with Fyn SH3 (20 µg/ml overnight, 100 µl per well). Blood samples (ranging from 75-200 µl) of each of the 5 mice were applied in dilution series (from 1:4 to 1:100). Detection of antibodies was performed using anti-mouse-IgG-HRP antibody conjugate (Sigma). As a control of the coating efficiency anti-HIS-HRP-conjugates (Sigma) were used.
   B) MaxiSorp plates (Nunc) were coated with Fyn SH3 D3 (20 µg/ml overnight, 100 µl per well), Blood samples (ranging from 76-200 µl) of each of the 5 mice were applied in dilution series (from 1:4 to 1:100). Detection of antibodies was performed using anti-mouse IgG-HRP antibody conjugate (Sigma). As a control of the coating efficiency anti-HIS-HRP-conjugates (Sigma) were used.
   C) MaxiSorp plates (Nunc) were coated with scFv (60 µg/ml overnight, 100 µl per well). Blood samples (ranging from 75-200 µl) of each of the 4 mice were applied in dilution series (from 1:4 to 1:100). Detection of antibodies was performed using anti-mouse-IgG-HRP antibody conjugate (Sigma). As a control of the coating
      efficiency, anti-myc-HRP-conjugates (Roche) were used.

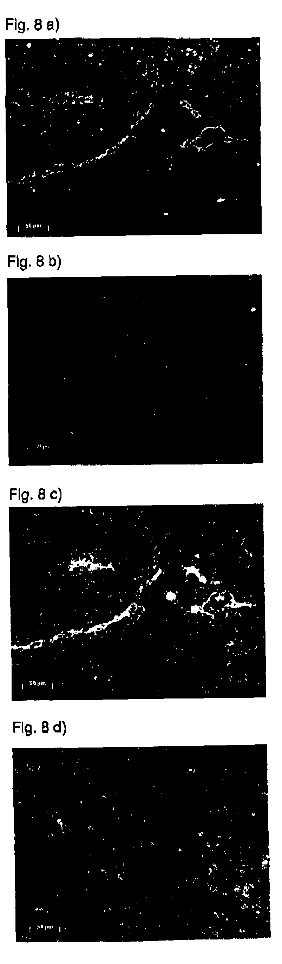

**Fig. 8** shows immunofluorescence of D3 (Fig. 8a), the corresponding negative control (8.b), the antl-CD31 staining (Fig. 8.c) and the corresponding negative control (8.d) on F9 murine teratocarcinoma histological sections.

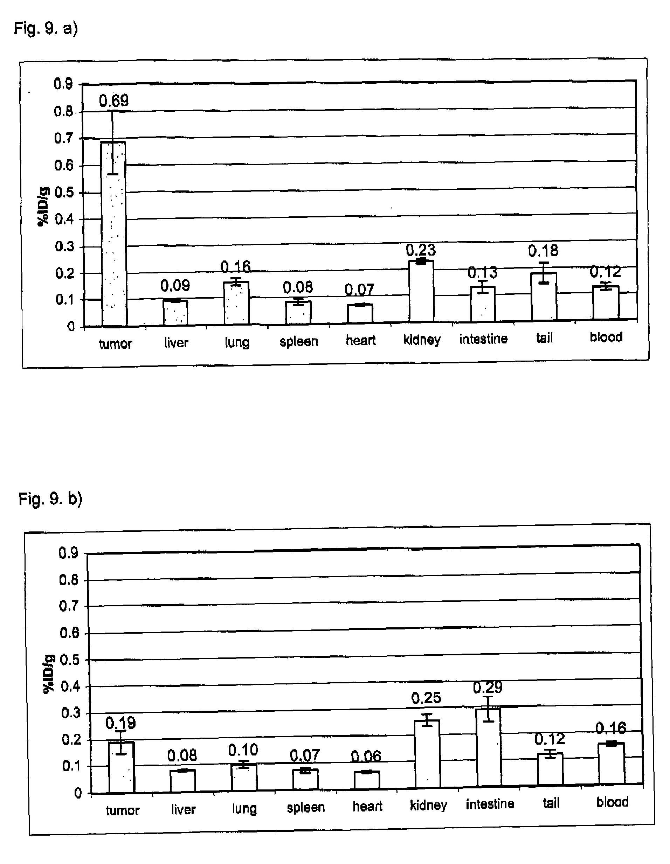

**Fig. 9** shows the tumor retention of Fyn SH3-D3 (Fig. 9. a)), whereas no accumulation could be observed for Fyn SH3wt (Fig. 9.b)) Targeting results are expressed as % injected dose of ¹²⁵I-labeled protein retained per g of tissue (%ID/g).

In the following the subject-matter of the Invention will be described in more detail referring to specific embodiments which are not intended to be construed as limiting to the scope of the invention.

### Examples

### Example 1: Expression of Fyn SH3 mutants

For the purpose of evaluating the expression of mutants of Fyn SH3 a dot blot analysis of three different Fyn SH3 sublibraries was performed (Fig.1): in the first library only the RT-loop was randomized, in the second the Src loop was randomized and extended to 6 residues and in the third library the RT- and the Src loop were randomized simultaneously, the latter loop being extended from 4 to 6 residues. The percentage of expressed Fyn SH3 mutants ranged from 59-90%.

**Table 1.**

| Library | Expressed mutants (%) | Number of clones tested |
|---|---|---|
| RT-Src | 59 | 29 |
| n-Src | 90 | 29 |
| RT-Src and n-Src | 62 | 58 |

### Example 2: Phage display selections against mouse serum albumin

A library of 10⁷ different Fyn SH3 was created (only the RT-loop was randomized) and cloned Into the phagemid vector pHEN1 (Hoogenboom et al. "Multi-subunit proteins on the surface of filamentous phage: methodologies for displaying antibody (Fab) heavy and light chains", Nucleic Acids Res, 19(15):4133-7, 1991). The library was displayed on phages and 3 rounds of panning were performed against mouse serum albumin (MSA), After the third round, screening for binding proteins was performed by monoclonal phage-ELISA; 13 positive clones were detected (Fig.2). Sequencing of the 13 clones revealed that two different sequences were enriched, denoted G4 and C4.

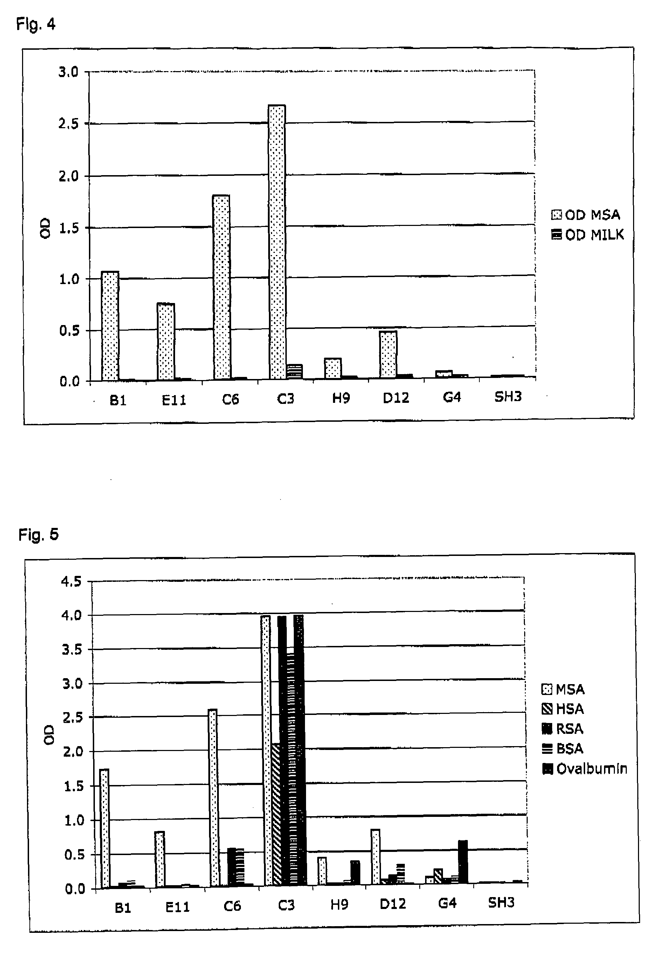

However, after subcloning and expression of G4 in the pQE-12 vector (Qiagen, expression and purifcation according to manufacturer's handbook under native conditions) the binding of the protein towards MSA could not be detected by ELISA (Fig. 4) due to low affinity (phage ELISA is more sensitive than the ELISA of the soluble protein). Therefore, the sequence of G4 was used for two different affinity maturation libraries (size: 10⁷ clones for each library). In the first one, the 4 residues of the n-Src loop and residues Trp37 (SEQ ID NO: 1) and Tyr50 (SEQ ID NO; 1) were randomized, in the second one the n-Src loop was extended from 4 to 6 randomized residues, After one round of panning several clones of both sublibraries gave stronger signals in Phage ELISA compared to the parental clone G4 (Fig.3). After subcloning and expression of several clones the binding of the soluble protein was confirmed by ELISA (Fig.4). Apparent dissociation constants were in the range of 100 nM (determined by BIAcore). Some of the clones were cross-reactive with other serum albumins (tested: human serum albumin (HSA), rat serum albumin (RSA), bovine serum albumin (BSA) and ovalbumin), whereas other clones were highly specific to MSA, indicating that it is possible to isolate high specific binding proteins (Fig.5).

### Example 3: Phage display selections against the extra domain b of fibronectin (ED-B)

ED-B was chosen as a target protein In order to demonstrate the ability to select Fyn SH3 derived binders against a pharmaceutically relevant protein. ED-B is a 91 amino acid Type III homology domain that is inserted Into the fibronectin molecule by a mechanism of alternative splicing at the level of the primary transcript whenever tissue remodelling takes place (Zardi et al., "Transformed human cells produce a new fibronectin isoform by preferential alternative splicing of a previously unobserved exon." Embo J. 6(8): 2337-42, 1987). It is a good quality marker of angiogenesis that is overexpressed In a variety of solid tumors (e.g. renal cell carcinoma, colorectal carcinoma, hepatocellular carcinoma, high-grade astrocytomas, head and neck tumours, bladder cancer, etc.) but is virtually undetectable in normal adult tissue (except for the endometrium in the proliferative phase and some vessels in the ovaries). (For more details on ED-B as a target see Menrad and Menssen, "ED-B fibronectin as a target for antibody-based cancer treatments." Expert Opin. Ther. Targets 9(3): 491-500, 2005).

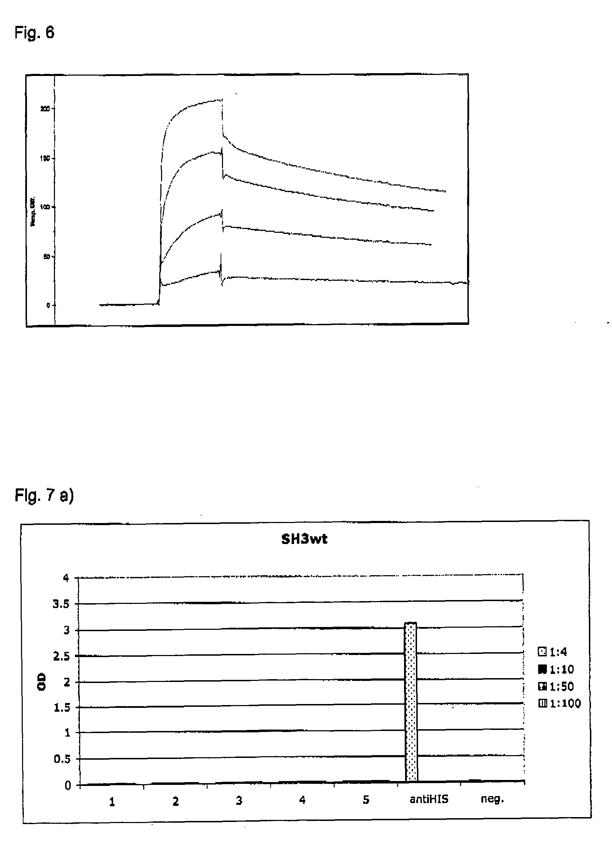

A library of more than 1 billion Fyn SH3 mutants was prepared and displayed on phages (simultaneous randomization of RT-Src and n-Src loops). After three rounds of panning against ED-B 3 binding clones were identified by phage ELISA. Sequencing revealed two different sequences (clones denoted B11 and D3). The dissociation constant of D3 was determined by surface plasmon resonance real-time interaction analysis using a BIAcore3000 instrument and showed a value of 8.5 x 10⁻⁸ M (Figure 6).

D3 (SEQ ID NO: 3)

GVTLFVALYDYHAQSGADLSFHKGEKFQILKFGRGKGDWWEARSLTTGETGYIPSNYV
APVDSIQ

### Example 4: Immunogenicity

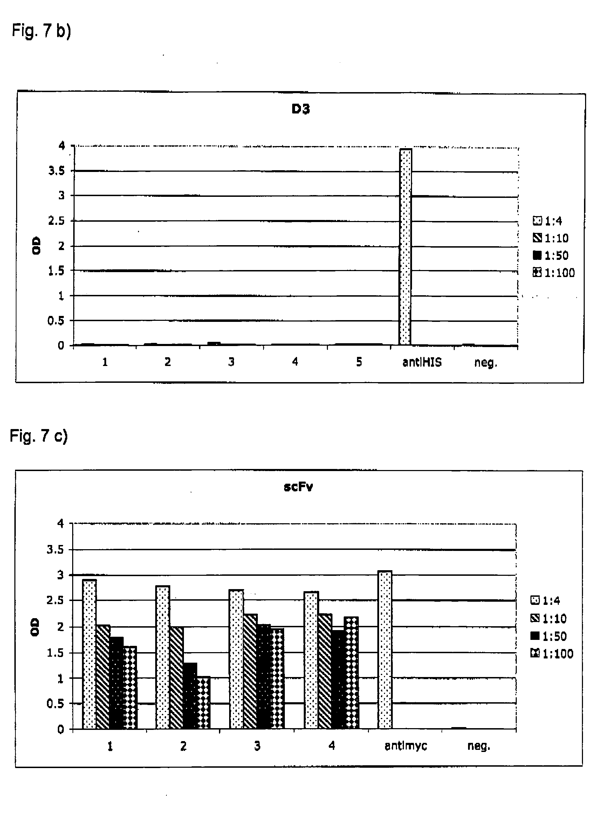

Immunogenicity of proteins is one of the major drawbacks in protein-related therapies, especially for treatments Involving repetitive administrations of a drug. Due to the conservation of the Fyn SH3 sequence in mice and men the immunogenic potential of the FynSH3 wild type protein (Fyn SH3wt) and a Fyn SH3 mutant (Fyn SH3D3, a binder against ED-B) was Investigated *in vivo* by injecting 5 mice repeatedly with the two proteins. Mice were injected 4 times (every third day) with 20 µg of protein. One day after the 4^{th} injection mice were sacrificed and blood samples were taken for examining the presence or absence of murine anti-Fyn SH3wt and anti-Fyn SH3D3 antibodies. As a positive control 4 mice were injected (equal time points of injection and equal dosages (= 60 µg)) with a human antibody in the single chain Fv format (scFv). However, one mouse of the scFv group died 20 minutes after the third injection and the other 3 were about to die, so blood samples were already taken after the third injection. Figures 7 a and b demonstrate that there were no detectable antibodies against Fyn SH3wt and Fyn SH3D3, whereas strong signals were observed for the control group (Fig. 7c).

### Example 5: Immunohistofluorescence

In order to explore whether Fyn SH3-D3 (D3, a binder against ED-B) recognizes its target in the native conformation in the tissue, immunofluorescence on F9 teratocarcinoma sections was performed. Figure 8 illustrates that D3 bound the tumor stroma around blood vessels (Fig. 8.a). The detection was performed with anti-His-Alexa488 antibody conjugate. In the negative control, no D3 protein was added (Fig. 8b). In order to visualize blood vessels, the same sections were co-stained with a rat anti-mouse-CD31 antibody and as a secondary antibody donkey anti-rat Alexa594 conjugate was used (Fig. 8.c). The negative control was done using the secondary antibody without the primary antibody (Fig. 8.d).

### Example 6: Quantitative biodistribution in vivo

The *in vivo* targeting performance of Fyn SH3-D3 (a binder against ED-B) and Fyn SH3 wild type (a non-binder to ED-B) was evaluated by biodistribution experiments in mice bearing a s,c, grafted F9 murine teratocarcinoma. Since ED-B is identical in mouse and man the results of the tumor targeting studies should be predictive of the D3 performance in humans. ¹²⁵I-labeled D3 and SH3wt were injected i.v. and 24h later, animals were sacrificed, the organs excised, weighed and radioactivity was counted. Figure 9.a shows that D3 selectively accumulated In the tumor (tumor:organ ratios ranged from 3:1 to 10:1), whereas no enrichment could be observed for the Fyn SH3 wild type protein (Fig. 9.b).

## Claims

1. A recombinant binding protein, comprising at least one derivative of the Src homology 3 domain (SH3) of the FYN kinase, wherein
(a) at least one amino acid in or positioned up to two amino acids adjacent to the src loop and/or
(b) at least one amino acid in or positioned up to two amino acids adjacent to the RT loop,
is substituted, deleted or added, wherein the SH3 domain derivative has an amino acid sequence having at least 70, preferably at least 80, more preferably at least 90 and most preferred at least 95 % sequence identity to the amino acid sequence of SEQ ID NO: 1,
with the proviso that the recombinant binding protein does not comprise the amino acid sequence of SEQ ID NO: 2,
and that the recombinant protein is not a natural SH3 domain containing protein existing in nature.

2. The binding protein according to claim 1, wherein said SH3 domain derivative has at least 85, preferably at least 90, more preferably at least 95, most preferably at least 98 to 100 % identity to the Src homology 3 domain (SH3) of the FYN kinase outside the src and RT loops.

3. The binding protein according to claim 1 or 2, wherein
(a) at least one amino acid in the src loop and
(b) at least one amino acid in the RT loop,
is substituted, deleted or added.

4. The binding protein according to any one of claims 1 to 3, comprising at least two derivatives of the SH3 domain, preferably a bivalent binding protein.

5. The binding protein according to any one of claims 1 to 4, comprising one or preferably two altered residues In positions 37 and/or 50 of the SH3 domain derivative, preferably two hydrophobic altered residues, more preferably Trp37 and/or Tyr50, Trp37 and Tyr50 being most preferred.

6. The binding protein according to any one of claims 1 to 5 having a specific binding affinity to a target of 10⁻⁷ to 10⁻¹² M, preferably 10⁻⁸ to 10⁻¹² M, preferably a therapeutically and/or diagnostically relevant target, more preferably an amino acid-based target comprising a PxxP motif.

7. The binding protein according to any one of claims 1 to 6 having a specific binding affinity of 10⁻⁷ to 10⁻¹² M, preferably 10⁻⁸ to 10⁻¹² M, to the extracellular domain of oncofetal fibronectin (ED-B).

8. The binding protein according to claim 7 having one or more, preferably two, altered, preferably hydrophobic, residues in positions 37 and/or 50 of the SH3 domain derivative, more preferably Trp37 and/or Tyr50, and most preferred Trp37 and Tyr50.

9. The binding protein according to any one of claims 1 to 8, comprising the amino acid sequence of SEQ ID NO: 3.

10. The binding protein according to any one of claims 1 to 9, wherein said binding protein has binding specificity for a protein or a small organic compound.

11. A fusion protein comprising a binding protein according to any one of claims 1 to 10 fused to a pharmaceutically and/or diagnostically active component.

12. The fusion protein according to claim 11, wherein said component is a cytokine, preferably a cytokine selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta.

13. The fusion protein according to claim 11, wherein said component is a toxic compound, preferably a small organic compound or a polypeptide, preferably a toxic compound selected from the group consisting of calicheamicin, neocarzinostatin, esperamicin, dynemicin, kedarcidin, maduropeptin, doxorubicin, daunorublein, auristatin, Ricin-A chain, modeccin, truncated Pseudomonas exotoxin A, diphtheria toxin and recombinant gelonin.

14. The fusion protein according to claim 11, wherein said component is a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MPIF-1, 6Ckine, CTACK, MEC, Lymphotactin and Fractalkine.

15. The fusion protein according to claim 11, wherein said component is a fluorescent dye, preferably a component selected from Alexa Fluor or Cy dyes.

16. The fusion protein according to claim 11, wherein said component is a photosensitizer, preferably bis(triethanolemine)Sn(IV) chlorine₆ (SnChe₆),

17. The fusion protein according to claim 11, wherein said component is a pro-coagulant factor, preferably tissue factor.

18. The fusion protein according to claim 11, wherein said component is an enzyme for pro-drug activation, preferably an enzyme selected from the group consisting of carboxy-peptidases, glucuronidases and glucosidases.

19. The fusion protein according to claim 11, wherein said component is a radionuclide either from the group of gamma-emifting isotopes, preferably ^{99m}Tc, ¹²³I, ¹¹¹In, or from the group of positron emitters, preferably ¹⁸F, ⁶⁴Cu, ⁶⁸Ga, ⁸⁶Y, ¹²⁴I, or from the group of beta-emitter, preferably ¹³¹I, ⁹⁰Y, ¹⁷⁷Lu, ⁶⁷Cu, or from the group of alpha-emitter, preferably ²¹³Bi, ²¹¹At.

20. The fusion protein according to claim 11, wherein said component is a functional Fc domain, preferably a human functional Fc domain.

21. The fusion protein according to any one of claims 11 to 20, further comprising a component modulating serum half-life, preferably a component selected from the group consisting of polyethylene glycol (PEG), immunoglobulin and albumin-binding peptides.

22. The fusion protein according to any one of claims 11 to 21, comprising the binding protein according to claim 7, 8 or 9.

23. A polynucleotide coding for a binding protein or fusion protein according to any one of claims 1 to 22.

24. A vector comprising a polynucleotide according to claim 23.

25. A host cell comprising a polynucleotide according to claim 23 and/or a vector according to claim 24.

26. A method for co-crystallizing proteins, wherein a binding protein according to any one of claims 1 to 10 is brought into contact with a second protein under conditions that allow for the crystallization of both proteins.

27. A method for purifying and/or enriching proteins, wherein a binding protein according to any one of claims 1 to 10 is contacted with a target protein under conditions that allow for the binding of the binding protein to the target protein.

28. Use of a binding or fusion protein according to any one of claims 1 to 14, 16 to 18 and 20 to 22 for preparing a medicament.

29. Use of a binding protein according to claim 7, 8 or 9 and/or a fusion protein according to claim 22 for preparing a medicament for the treatment of cancer.

30. Use of a binding or fusion protein according to any one of claims 1 to 10, 15, 19, 21 and 22 for preparing a diagnostic means.

31. Use of a binding protein according to claim 7, 8 or 9 and/or a fusion protein according to claim 22 for preparing a diagnostic means for the diagnosis of cancer.

32. A pharmaceutical composition comprising a binding or fusion protein according to any one of claims 1 to 14, 16 to 18 and 20 to 22 and optionally a pharmaceutically acceptable excipient.

33. A diagnostic composition comprising a binding or fusion protein according to any one of claims 1 to 11, 15, 19, 21 and 22 and optionally a pharmaceutically acceptable excipient.
